# EUROPEAN PATENT APPLICATION

(11) **EP 1 702 619 A2**
(43) Date of publication of application: **20.09.2006**
(21) Application number: 06006547.1
(22) Date of filing: 29.04.2002
(51) Int. Cl.: A61K 31/573, A61K 9/08, A61K 47/48, A61K 31/57

(54) **Disinfecting and solubilizing steroid compositions**

(30) Priority: 07.05.2001 US 289337 P
(62) Divisional of application: 02769298.7
(71) Applicant: ALLERGAN, INC., Irvine, CA 92612 (US)
(72) Inventor: Lyons, Robert, T., Laguna Hills, CA 92653-7533 (US)
(74) Representative: HOFFMANN EITLE

(57) **Abstract**

A method of making an inclusion complex of prednisolone or prednisolone acetate and a cyclodextrin or cyclodextrin derivative comprising ultrasonicating a solution comprising the cyclodextrin or cyclodextrin derivative, prednisolone or prednisolone acetate, and the water-soluble polymer.

## Description

This application claims priority under 35 U.S.C. §119(e)(1) to provisional application number 60/289,337, filed May 7, 2001, which is hereby incorporated by reference herein.

### Background of the Invention

The eye, like other parts of the central nervous system, has limited regeneration capability. Thus, many ocular diseases and injuries are difficult to treat. Presently, there are no truly effective treatments for, for example, retinal photic injury, retinal ischemia-induced eye injury, age-related macular degeneration, and free-radical-mediated diseases and/or injuries. Certain of these degenerations and injuries result in the irreversible destruction of the photoreceptor cells; therefore prophylaxis is the only viable option for management. Loss of vision also arises as a result of ischemia-reperfusion injury that is associated with retinal arterial occlusion, retinal venous occlusion, and glaucoma.

Many ocular degenerations are secondary to other primary compromising conditions, for example, diabetic retinopathy and lupus retinopathy. Corneal degenerations, for example, are usually not inherited, but occur in mid-life or later with lesions that are secondary to primary manifestations of aging, inflammation, trauma, and systemic disease.

The eye is also particularly vulnerable to infection caused by virulent bacteria. The most frequently encountered bacterial infections are believed to be bacterial keratitis, bacterial conjunctivitis, and bacterial blepharitis. The most significant ocular viral infections are caused by the family of herpesviruses (HSV-1, HSV-2, varicella-zoster virus, cytomegalovirus, and Epstein-Barr virus.) Some ocular tissues (e.g., cornea, lens, and vitreous) are avascular with few mesenchymal cells and therefore are highly susceptible to infection. Ocular tissue already compromised due to degenerative injury (e.g., lesions) or physical trauma (e.g., laceration) affords easy entrance to bacteria and viruses. For example, infection can follow superficial or penetrating corneal injury, and the type of offending matter and the time between trauma and therapy are oftentimes determinative of the type and extent of infection. Fungal infection can be seen in surface injuries involving vegetable matter. Another competing consideration is the fact that certain therapeutic agents used to treat ocular injury and/or infection also suppress the host's immunologic defense mechanism, thus rendering the eye susceptible to other types of infections.

Ocular inflammation is a nonspecific result of tissue damage. While there are several agents that can elicit an inflammatory response, microbial (bacterial, viral, or fungal) infection and various immunologic conditions (e.g., hypersensitivity, allergy, and autoimmunity) are the most common causes of ocular inflammation. Inflammation associated with chemical and thermal injury can have a highly destructive outcome on the eye, and especially the cornea. Physical trauma to the cornea may be accompanied by intraocular inflammation, synechiae leading to glaucoma, and secondary membrane formation. Collagen is the major structural protein of the cornea. The normal host response to inflammation produces polymorphonuclear (PMN) leukocytes or corneal fibroblasts which release matrix-destroying enzymes (e.g., collagenases), leading to the destruction of collagen. Also, normal corneal epithelium contains no latent or active collagenases. However, following chemical injury to the eye, these cells have been known to produce the destructive enzyme. Other macromolecules such as proteoglycans and other glycoproteins are also destroyed. Neovascularization is a sequela to the majority of ocular inflammatory responses. Chronic ocular inflammations such as trachoma and inflammation resulting from penetrating corneal injuries lead to scarring of the cornea. This is attributable to the enhanced production of collagen by corneal and conjunctival tissue fibroblasts as potentiated by the presence of inflammatory cells. Stromal scarring (e.g., from stromal edema) disturbs the ordering and spacing of collagen fibrils that are necessary to prevent light scattering, and causes a loss of stromal transparency.

The inflammatory response is a dominant aspect of corneal ulceration (ulcerative keratitis), which is a frequent cause of vision loss. Corneal ulceration has several causes, chiefly viral (e.g., Herpes simplex is the most common and is the leading cause of corneal blindness in the U.S.) or bacterial infection (*Pseudomonas* sp.), chemical (e.g., alkali burn) and thermal injury, and vitamin A and protein deficiencies. Enzymatic breakdown of collagen is the major degenerative aspect of the ulceration. The outcome of ulceration, it untreated, is one or more of perforation of the cornea, formation of opaque scar tissue, and vascular invasion, with ultimate blindness. The inflammatory response is also at work in the corneal stroma in nonulcerative keratitis (also, interstitial keratitis), which has either bacterial, viral, or parasitic origin. Although less frequent than ulcerative bacterial keratitis, interstitial keratitis is a significant cause of visual impairment in developing countries, and the major causes of which are *T. pallidum* (the syphilis bacteria) and Borrelia *burgdorferi* (Lyme disease.)

Refractive surgical procedures aimed at altering corneal curvature for treating myopia and astigmatism, for example, result in a disruption of several corneal components, such as epithelial cells and their adhesion structures, the Bowman's layer and the anterior stroma. Incisional procedures (e.g., radial keratotomy (RK)) utilizing cutting implements invariably damage many layers of cells adjacent to the incision, and hence impair the wound-healing ability without attendant scar formation. The use of UV and non-UV emitting lasers in ocular surgery (e.g., excimer laser keratectomy, photorefractive keratectomy (PRK) and laser in-situ keratomileusis (LASIK)) has evolved to minimize the extent of cell disruption during excisional procedures and to enhance the wound-healing ability of the surgical site. However, despite the improvements of lasers over cutting implements, one of the main drawbacks of corrective laser procedures is the development of "corneal haze", or clouding, leading to light scattering. While many reasons have been postulated as to why the haze develops, the chief theory is that the haze is a scar resulting from improper wound healing. Improper collagen repair and/or alignment, inflammation, and improper epithelial cell coverage of the cornea are believed to play a role in the scar formation. Another drawback of laser procedures is that they set into motion a cascade of free-radical mediated cellular injuries, such as DNA damage, enzyme inactivation, and lipid peroxidation, leading to corneal toxicity which may impact on wound healing and the development of post-operative corneal haze.

Numerous therapies and therapeutic agents have been developed over the years to treat sequelae of ocular degeneration, physical and chemical traumatic ocular injury, and ocular inflammation. While many of these have proven to be useful and provide an acceptable level of therapy and reparation to the damaged eye tissue, others have unacceptable side effects that dispose the already impaired/injured eye to further vulnerability (e.g., toxicity.) For example, corticosteroids have been used topically to reduce corneal scarring and inflammation. However their use is deemed controversial because they are known to enhance bacterial growth or recurrence of ulcers. Many antibiotics (e.g., beta-lactams and certain fluoroquinolones) are not well-tolerated, give rise to toxicities, or are of moderate efficacy.

The use of immunosuppressive agents in treating autoimmune ocular disease, e.g., uveitis, is controversial because of many serious side effects including bone marrow depression, thrombocytopenia, bleeding, nausea, vomiting, and stomatitis occur. Without attempting a comprehensive and exhaustive list of agents that have proven beneficial in the management of primary and secondary sequelae of ocular degeneration, injury, surgical trauma, and attendant inflammation, representative classes of compounds include antibacterials(e.g., broad spectrum antibiotics), antivirals, non-steroidal antiinflammatory agents, steroids, collagenase inhibitors, cholinergics, cycloplegics, and wound healing modulators.

Among the agents which have been shown to have efficacy in the treatment of ocular inflammation are steriods including, without limitation, dexamethasone, prednisolone, prednisone, fluorometholone, betamethasone, and hydrocortisone. Many compounds having utility as therapeutic compounds for the treatment of ocular conditions, including many steriods, are hydrophobic compounds having little solubility in aqueous solution at roughly neutral pH values. While some such compounds have been formulated at pH values above or below the range from about 6.8 to about 7.8 in order to cause any ionizable groups to become charged, ophthalmic solutions or suspensions formulated at such values are usually irritating to the patient. Additionally, the resulting charged agent is less able to permeate the corneal epithelium than its uncharged counterpart, and is therefore less effective in delivering its therapeutic effect.

Methods for increasing the solubility of hydrophobic drugs have typically involved formulating the drug either as a suspension or in an emulsion. Given the short residence time of topically applied ophthalmic solutions, suspensions are of limited usefulness in that they require the preparation of a saturated solution of the drug in which the compound in suspension cannot dissolve until the temperature of the solution increases or in which loss of the drug from solution by transport across the corneal epithelium permits more solid drug to dissolve. As both of these results take some time, the amount of solution lost in the meantime through tearing and by drainage through the lacrimal and naso-lacrimal ducts can be considerable and lead to decreased bioavailability of the therapeutic agent.

Emulsions comprise either oil-in-water or water-in-oil systems in which the hydrophobic therapeutic agent is dissolved in lipid globules suspended in an aqueous phase, or in an oil phase which surrounds suspended droplets of the aqueous phase, respectively. A common problem with most emulsions for topical ocular delivery of a therapeutic agent is that, they can cause ocular irritation and blurred vision for a time following application.

Relatively recently members of a class of barrel-shaped cyclic oligosaccharides called cyclodextrins have been shown to improve the physiochemical properties of certain drugs through the formation of inclusion complexes. Cyclodextrins (CDs) consist of 6, 7 or 8 glucose units; these cyclodextrins are termed alpha, beta or gamma cyclodextrins, respectively. Due to the architecture of the cyclodextrin molecule, the interior of the "barrel" is hydrophobic, which the exterior of the molecule is ionic. In certain cyclodextrin derivatives one or more glucose units may be substituted with various groups, such as hydroxypropyl (HP) groups or sulfobutylether (SBE) groups. Such substitutions are usually found in the exterior of the CD molecule.

CDs have been shown to increase the aqueous solubility and stability of poorly water soluble drugs. See Loftssona et al., *Advanced Drug Delivery Reviews* 36:59-79 (1999); this and all references cited herein are hereby incorporated by referenced as part of this specification unless specifically excluded. Thus, the aqueous stabilities of the drugs pilocarpine, cetirizine, hydrocortizone and dexamethasone has been shown to have been increased by formulation of these drugs in combination with cyclodextrin derivatives.

Sequesterization of the drug within the barrel of the cyclodextrin molecule increases the solubility of the drug, however, therapeutic efficacy requires that the drug also be released from the CD effectively enough to permit the drug's passage through the corneal epithelium, since the CD-drug complex does not appear to permeate the cornea itself. For example, experimental evidence has demonstrated that complexation of pilocarpine with SBE4-β-CD, despite increasing solubility of pilocarpine in aqueous solution, renders the complex unable to penetrate the cornea. *Id*. at 70.

The amount of CD used for complexation must be kept as low as possible for toxicological, tonicity and bioavailability reasons. The use of water-soluble, therapeutically inert polymers such as polyvinylpyrrolidone (PVP) and cellulose derivatives such as hydroxypropylmethylcellulose (HPMC) as an aid in enhancing complex formation has been disclosed. See *id.;* see also US Patent No. 5,324,718. Such enhancement of complex formation means that less CD can be used for the ophthalmic formulation ultimately used.

Optimal enhancement of complex formation using CD and polymers appears to require the application of heat at temperatures of 120° C or more. However, the very heat used to enhance solubility and complex formation can result in degradation and loss of stability of the drug. Thus, the drug is often added after the CD-polymer complex is formed, even though this results in an additional step in the process. See US Patent No. 5,324,718.

For these reasons new methods for preparing ophthalmic CD-polymer-drug complexes are needed. Once such method would protect and stabilize the active drug during exposure to high temperatures. Another such method would provide a high efficiency method of complex formation without the application of heat. Also, new self-stabilizing therapeutic compositions would be useful which compositions preserve the active drug while being complexed with CD and a water soluble polymer during autoclaving or other exposure to high temperature.

### Summary of the Invention

The present invention is drawn to methods and compositions for stabilizing, solubilizing, and increasing bioavailability of a drug having low aqueous stability. In one embodiment the drug is formulated as an topical ophthalmic solution having increased comfort and able to delivery the active drug so as to effectively provide a therapeutic effect. In a preferred embodiment the active drug is a steroid; in a particularly preferred embodiment the steroid is prednisolone.

The claimed ophthalmic compositions utilize cyclodextrins or cyclodextrin derivatives in complex with a drug, a water soluble polymer such as a cellulose derivative (e.g., methyl cellulose, hydroxypropylmethylcellulose) and a cationic buffer. Preferably the cationic buffer is an amine buffer and has a pKa in the slightly acidic range, e.g., about pH 5.0 to about 7.0, even more preferably about 6.0. The buffer is preferably selected from histidine or bis-tris buffers. Such a composition is capable of being formulated as a drug-CD inclusion complex at high heat with significantly reduced degradation and loss of stability than when the drug is formulated in an anionic buffer, such as phosphate buffer.

In another embodiment, the invention comprises forming such inclusion complexes by the ultrasonication of a solution comprising cyclodextrin, drug and an optional water soluble polymer. Such complexation can be done without the use of high heat, such as that provided by autoclaving. In this case any suitable common buffer (other than phosphate buffers) can be used, regardless whether they are cationic or anionic in the ionized form.

### DETAILED DESCRIPTION OF THE INVENTION

In one embodiment the present invention is drawn to methods for the formulation of lipophilic drugs for ophthalmic topical delivery using cyclodextrins as an aid to solubilizing such drugs in aqueous solution. Without limitation, such drugs may be chosen from those lipophilic drugs contained in the following listing: ciprofloxacin, ofloxacin, norfloxacin, cefazolin, tobramycin, gentamycin, an aminoglycoside, a penicillin, a semi-synthetic penicillin, amoxicillin, ampicillin, carbenicillin, ticarcillin, mezlocillin, a cephalosporin, vancomycin, chloramphenicol, erythromycin, clindamycin, rifampin, bacitracin, polymyxin, spectinomycin, a sulfonamide, trimethoprim, super oxide dismutase, astaxanthin, canthazanthin, beta-carotene, zeaxanthin, lutein, alpha-tocopherol, ascorbic acid, glutathione, selenous acid, sodium selenate, acyclovir, ganciclovir, idoxuridine, vidarabine, trifluridine, bromovinyldeoxyuridine, azidothymidine, amantadine, rimantadine, dexamethasone, prednisolone, prednisone, fluorometholone, betamethasone, hydrocortisone, an alpha-hydroxyacid, a beta-hydroxyacid, an alpha-ketoacid, a beta-ketoacid, ketorolac, indomethacin, flurbiprofen, loxoprofen, diclofenac, atropine, pilocarpine, carbachol, physostigmine, phenylephrine, acetazolamide, timolol maleate, fibronectin and vitronectin as well as analogs or fragments thereof, acetyl cysteine, or mixtures thereof.

The cyclodextrins may be selected from naturally occurring cyclodextrins or their synthetic derivatives. Cyclodextrins are cyclic oligosaccharides with hydroxyl groups on the outer surface and a void cavity in the center. Their outer surface is hydrophilic, and therefore they are usually soluble in water, but the cavity has a lipophilic character. The most common cyclodextrins are α-cyclodextrin, β-cyclodextrin and y-cyclodextrin, consisting of 6, 7 and 8 α-1,4-linked glucose units, respectively. The number of these units determines the size of the cavity.

Some common cyclodextrin derivatives are, without limitation, formed by alkylation (e.g. methyl- and ethyl-β-cyclodextrin) or hydroxyalkylation of the hydroxyl groups (e.g. hydroxypropyl- and hydroxyethyl-derivatives of α-, β-, and γ-cyclodextrin) or by substituting the primary hydroxyl groups with saccharides (e.g. glucosyl- and maltosyl-β-cyclodextrin). Hydroxypropyl-β-cyclodextrin and its preparation by propylene oxide addition to β-cyclodextrin, and hydroxyethyl-β-cyclodextrin and its preparation by ethylene oxide addition to β-cyclodextrin, were described in a patent of Gramera et al. (U.S. Pat. No. 3,459,731, issued Aug. 1969) over 20 years ago. Cycodextrin is normally present at a concentration of about 10% to about 30% by weight.

In certain embodiments the invention comprises, either optionally or as a mandatory component, a water soluble polymer as an aid in complex formation, present at from about 0.1% to about 5% by weight. Examples of such water soluble polymers include cellulose derivatives, polyvinyl pyrrolidone and the like. When formulated a drug-CD-polymer complex may be formed at high heat (e.g., by autoclaving) in a cationic buffer such as an amine buffer. Such buffer may comprise, without limitation, a histidine buffer or a bis-tris buffer. This is particularly useful when formulating a steroid such a prednilosone. Buffer concentrations are in the range from about 10 to about 50 mM, preferably about 20 mM.

In another embodiment the invention comprises a method for forming drug-cyclodextrin complexes, either with or without a polymer, by ultrasonication, preferably with a high-energy probe sonicator. For larger scale lots high pressure, high cavitation ultrasonic homogenizers are commercially available and may be used.

A further embodiment of the invention comprises the use of a boric acid/sodium borate buffer system in conjunction with stabilized chlorine dioxide (e.g., the form of stabilized chlorine dioxide sold under the trade name Purite® by Allergan, Inc.) to safely and effectively preserve and increase the shelf life of cyclodextrin-based drug formulations. See International Patent Application Publication No. WO 00/12137, incorporated by reference herein.

The following Examples do not limit, but rather illustrate the invention, which is defined solely by the claims that conclude this specification.

### EXAMPLES

### Example 1

To optimize a cyclodextrin-based formulation for the ocular administration of soluble prednisolone acetate (PA), the following methods were used to evaluate ophthalmic formulations of PA and methods of making such formulations.

The complexation of five β-cyclodextrin (CD) derivatives with PA was evaluated, both with and without added cellulose polymer (HPMC). The β-cyclodextrins were: methyl-β-cyclodextrin, HP-CD and SBE-CD, with the latter being substituted by an average of either 12, 7, or 4 groups per molecule.

Methods of making inclusion complexes were:(I) rapid stirring at 25°C for 72 hrs, (II) high-shear processing at 60°C with a rotor/stator homogenizer, (III) brief ultrasonication with a high-energy probe sonicator, and (IV) autoclaving in sealed borosilicate glass vials for 10 min at 121°C. In every case, an equimolar concentration of PA was added to 10% solutions of CD in dilute (20 mM) aqueous buffer prior to complex formation. After processing, aliquots were filtered (0.45µm) for HPLC analysis of soluble, complexed PA and the hydrolytic degradant, non-esterified prednisolone (P).

### The formulations were as follows:

| Ingredient | Grams / 100 mL |
|---|---|
| Cyclodextrin | 10.0 |
| HPMC | 0.5 |
| Prednisolone acetate | 0.5 |
| Boric Acid | 0.6 |
| Na borate | 0.035 |
| Purite | 0.005 |
| HCl | adjust to pH 7 |

Results were as follows. Among tested β-CD derivatives, methyl was by far the most efficient solubilizer (PA/CD molar ratio). Although only 40% as effective, hydroxypropyl (HP) had a superior toxicity profile. Affinity of sulfobutyl ether CD for PA increased as degree of substitution was reduced (12, 7, 4), but was never as high as HP.

Observed complexation efficiency for each method was as follows: IV > II = III > I. During autoclaving, complexation was enhanced by about 70% (to 4.6 mg/mL) in the presence of 0.1% hydroxypropylmethyl cellulose (HPMC), but not by other tested polymers. Autoclave stress allowed quick screening for buffer catalysis of PA hydrolysis. It was found that phosphate salts accelerated hydrolysis by about 16-fold compared to acetate buffer or no-buffer control.

### Example 2

Prednisolone acetate (PA) is solubilized with a 5% excess of either hydroxypropyl (HP) β-cyclodextrin (CD) or sulfobutyl ether 4 (SBE4) β-cyclodextrin in the presence of hydroxypropylmethyl cellulose (HPMC). SBE4, with an average molecular substitution of four, is the preferred derivative due to a higher binding capacity for PA and a lower contribution to ionic strength. HPMC serves both to increase solution viscosity and enhance stability of the drug-CD complex. In order to minimize rates of PA hydrolysis and maintain patient comfort, the solution is adjusted to pH 6 using a 20 mM histidine buffer system. Buffer salts with high electron density, such as phosphate, are avoided since these appear to catalyze PA hydrolysis.

A preferred formulation is as follows:

| Ingredient | Grams / 100 mL |
|---|---|
| Cyclodextrin | 10.0 |
| HPMC | 0.5 |
| Prednisolone acetate | 0.5 |
| Histidine (20 mM) | |
| PHMB (1 ppm) | |
| HCl | adjust to pH 6.0 |

This formulation is autoclaved in sealed borosilicate glass vials for 10 min. at 121°C to enhance complex formation, then cooled to room temperature before aliquots are taken for HPLC analysis of complexed drug and any degradation products.

The results indicate that this formulation is more effective at stabilizing the prednisolone and preventing degradation when complexes are formed at high temperature than the formulation of Example 1. Key elements are formulation at pH below about 7.0 and use of a cationic buffer, in this case histidine.

Other embodiments of the invention are disclosed in the following claims.

## Claims

1. A method of making an inclusion complex of prednisolone or prednisolone acetate and a cyclodextrin or cyclodextrin derivative comprising ultrasonicating a solution comprising the cyclodextrin or cyclodextrin derivative, prednisolone or prednisolone acetate, and the water-soluble polymer.

2. The method of claim 1, wherein the solution additionally comprises a buffer other than a phosphate buffer.

3. The method of claim 2 or 2, wherein said cyclodextrin derivative is an SBE cyclodextrin.

4. The method of claim 2, wherein said buffer is an amine buffer.

5. The method of claim 4, wherein said buffer is histidine.

6. The method of claim 5, wherein said buffer is bis-tris.

7. An aqueous ophthalmic composition comprising the inclusion complex obtainable by the method as defined in any one of claims 1 to 6 and formulated at a pH below 7.0.
